# EUROPÄISCHE PATENTSCHRIFT

(11) **EP 2 061 737 B1**
(45) Veröffentlichungstag und Bekanntmachung des Hinweises auf die Patenterteilung: **10.10.2012**
(21) Anmeldenummer: 07786661.4
(22) Anmeldetag: 13.08.2007
(51) Int. Cl.: C07B 41/10, C07C 231/02, C07C 233/30, C07C 231/14

(54) **VERFAHREN ZUR HERSTELLUNG VON ACYLGLYCINATEN MITTELS DIREKTOXIDATION**
PROCESS FOR PREPARING ACYLGLYCINATES BY MEANS OF DIRECT OXIDATION
PROCEDE DE PREPARATION D'ACYLGLYCINATES PAR OXYDATION DIRECTE

(30) Priorität: 18.08.2006 DE 102006038853
(43) Veröffentlichungstag der Anmeldung: 27.05.2009
(73) Patentinhaber: Clariant Finance (BVI) Limited, Road Town, Tortola (VG)
(72) Erfinder: KLUG, Peter, 63762 Grossostheim (DE); STANKOWIAK, Achim, 84503 Altötting (DE); FRANKE, Oliver, 84453 Mühldorf a. Inn (DE); SCHERL, Franz-Xaver, 84508 Burgkirchen (DE); PRÜSSE, Ulf, 38118 Braunschweig (DE); DECKER, Nadine, 38106 Braunschweig (DE); VORLOP, Klaus-Dieter, 38102 Braunschweig (DE)
(74) Vertreter: Paczkowski, Marcus
(86) Internationale Anmeldenummer: PCT/EP2007/007128
(87) Internationale Veröffentlichungsnummer: WO 2008/019807

(56) Entgegenhaltungen:
- EP-A- 1 314 717
- CHOJI KASHIMA ET.AL: "Amino alcohols as C-terminal protecting groups in peptide synthesis" J. CHEM. SOC. PERKIN TTANS I, Bd. 3, 1988, Seiten 535-539, XP009092666

## Beschreibung

Aminosäuretenside sind in der Waschmittel- und Kosmetikindustrie weit verbreitet. Sie zählen zu der Gruppe der milden Cotenside und werden meist zur Verbesserung des Schaumvolumens und der Milde der Formulierungen eingesetzt. Sie wurden in der Vergangenheit hauptsächlich über die Umsetzung von Aminosäuren mit aktivierten Fettsäurederivaten, speziell Fettsäurechloriden, synthetisiert wie z.B. in US 6,703,517 oder US 2005/0085651 A1 EP1 314 717 für Acylglycinate der Formel (IIa) beschrieben (s. Schema 1).

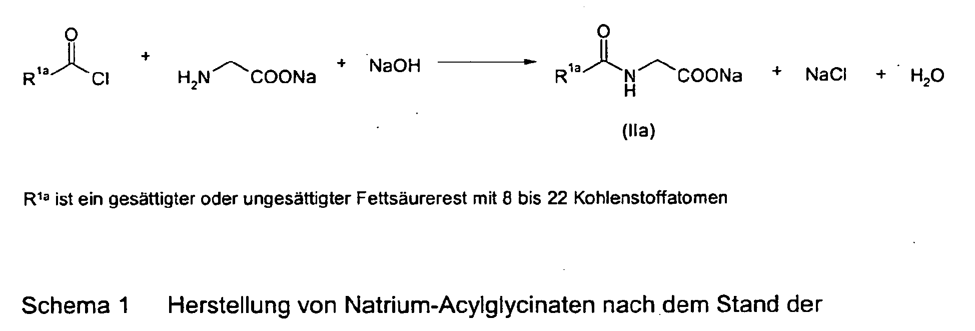

Dieses Verfahren nach dem Stand der Technik benötigt mit dem Fettsäurechlorid einen relativ teuren und reaktiven Rohstoff und weist außerdem den Nachteil auf, dass pro ein mol Aminosäuretensid, d.h. Verbindung der Formel (IIa), ein mol Kochsalz NaCl gebildet wird. Dieses gelangt in das Reaktionsabwasser und stellt dort für biologische Kläranlagen ein Problem dar, da Kochsalz die Reinigungsleistung solcher Anlagen beeinträchtigen kann.

Es bestand also ein Bedarf an Verfahren zur Herstellung von Aminosäuretensiden und hier speziell Aminosäuretensiden auf Basis der Aminosäure Glycin, so genannte Acylglycinate und deren protonierte Basissäuren, die die obigen Nachteile nicht aufweisen.

Überraschend wurde gefunden, dass acylierte Glycine und deren Salze, so genannte Acylglycinatsalze oder kurz Acylgylcinate, alternativ zu der üblichen Fettsäurechloridroute nach dem Stand der Technik auch durch direkte Oxidation von Fettsäuremonoethanolamiden mit Luftsauerstoff oder Reinsauerstoff mittels Nebengruppenmetallkatalysatoren zugänglich sind.

Gegenstand der vorliegenden Erfindung ist daher ein Verfahren zur Herstellung von Acylglycinatsalzen der Formel (II) 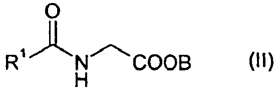 worin
- R¹: einen gesättigten linearen oder verzweigten Alkylrest mit 1 bis 21 Kohlenstoffatomen oder einen ein- oder mehrfach ungesättigten linearen oder verzweigten Alkenylrest mit 2 bis 21 Kohlenstoffatomen und
- B: ein Kation, das von einer Base abgeleitet ist,
bedeutet, und/oder der entsprechenden protonierten Acylglycinsäuren, dadurch gekennzeichnet, dass ein oder mehrere Fettsäuremonoethanolamide der Formel (I) 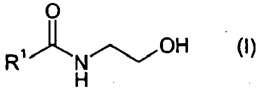 worin R¹ die oben angegebene Bedeutung besitzt,
mit Sauerstoff in Gegenwart eines Nebengruppenmetallkatalysators im alkalischen Medium zu einem oder mehreren Acylglycinatsalzen der Formel (II) oxidiert werden und im Fall der Herstellung der protonierten Acylglycinsäuren das oder die Acylglycinatsalze der Formel (II) zusätzlich mit einer Säure umgesetzt werden.

Bei dem erfindungsgemäßen Verfahren wird gegenüber der Verwendung des Fettsäurechlorids mit einem deutlich preisgünstigeren Rohstoff, dem Fettsäuremonoethanolamid der Formel (I), gestartet. Außerdem wird bei der Herstellung der Acylglycinate der Formel (II) kein Salz gebildet (s. Schema 2).

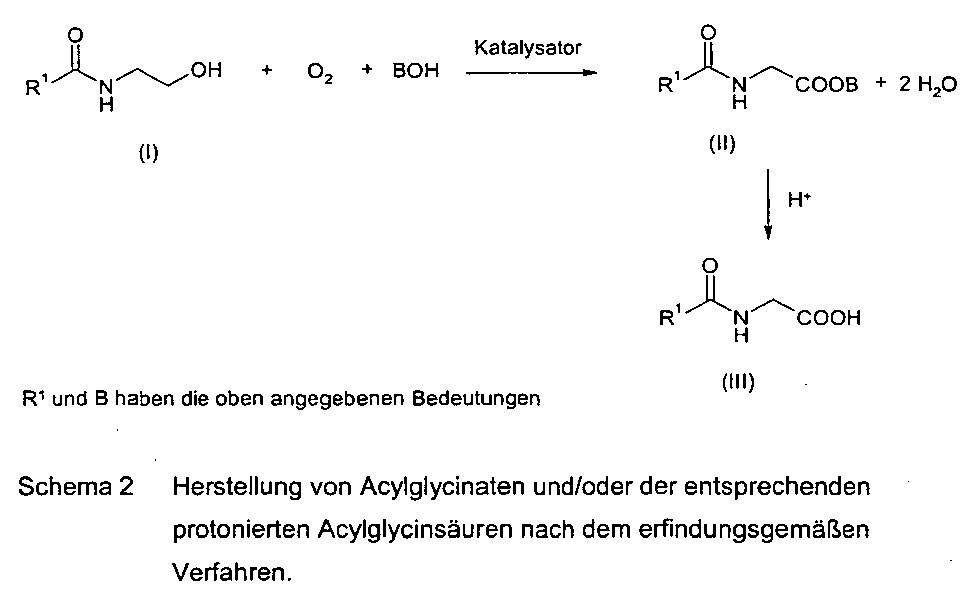

Als Fettsäuremonoethanolamide können Monoethanolamide von gesättigten, unverzweigten oder verzweigten Fettsäuren mit 2-22 Kohlenstoffatomen (d.h. R¹ = C₁ bis C₂₁) oder von ein- oder mehrfach ungesättigten, unverzweigten oder verzweigten Fettsäuren mit 3-22 Kohlenstoffatomen (d.h. R¹ = C₂ bis C₂₁) eingesetzt werden.

Bevorzugt sind Fettsäuremonoethanolamide mit 8-18 Kohlenstoffatomen im Fettsäurerest bzw. Acylrest R¹CO-, d.h. R¹ ist in diesem Fall ein gesättigter linearer oder verzweigter Alkylrest mit 7 bis 17 Kohlenstoffatomen oder ein ein- oder mehrfach ungesättigter linearer oder verzweigter Alkenylrest mit 7 bis 17 Kohlenstoffatomen. Besonders bevorzugt sind Laurinsäuremonoethanolamid, Myristinsäuremonoethanolamid, Caprylsäuremonoethanolamid, Caprinsäuremonoethanolamid, Palmitinsäuremonoethanolamid, Stearinsäuremonoethanolamid oder Isostearinsäuremonoethanolamid. Hierbei können auch Amide basierend auf Kettenschnitten bzw. Mischungen dieser Fettsäuremonoethanolamide eingesetzt werden, bevorzugt Cocosfettsäuremonoethanolamid.

Unter den gesättigten und ungesättigten Fettsäuremonoethanolamiden sind die gesättigten Fettsäuremonoethanolamide bevorzugt.

Bei den Gegenionen B handelt es vorzugsweise um Alkalimetallkationen ausgewählt aus Kationen der Alkalimetalle Li, Na, K, Rb und Cs. Besonders bevorzugt sind die Kationen der Alkalimetalle Na und K.

Als Nebengruppenmetallkatalysatoren kommen bevorzugt heterogene Edelmetallkatalysatoren in Frage. Geeignete Edelmetalle sind Kupfer, Silber, Gold, Ruthenium, Rhodium, Palladium, Osmium, Iridium, Platin oder deren Mischungen. Bevorzugt sind Gold-Katalysatoren.

Besonders bevorzugt ist der Nebengruppenmetallkatalysator ein Nano-Gold-Katalysator. Dies bedeutet, dass die Teilchengröße der Goldteilchen im Nanometer-Bereich angesiedelt ist.

Vorzugsweise sind die Edelmetalle auf Trägern aufgebracht. Bevorzugte Träger sind Aktivkohle und oxidische Träger, vorzugsweise Titandioxid oder Aluminiumoxid. Solche Katalysatoren können wie in L. Prati, G. Martra, Gold Bull. 39 (1999) 96 und S. Biella, G.L. Castiglioni, C. Fumagalli, L. Prati, M. Rossi, Catalysis Today 72 (2002) 43-49 oder L. Prati, F. Porta, Applied catalysis A: General 291 (2005) 199-203 beschrieben, hergestellt werden.

Besonders bevorzugt sind heterogene Goldkatalysatoren auf einem Träger, insbesondere bevorzugt solche mit nanofeinem Gold. Außerordentlich bevorzugt ist der Nano-Gold-Katalysator auf einem oxidischen Träger aufgebracht. Vorzugsweise besteht dieser Träger aus Titandioxid.

Die Nano-Gold-Katalysatoren enthalten vorzugsweise 0,1 bis 5 Gew.-% Gold und besonders bevorzugt 0,5 bis 2 Gew.-% Gold.

Die Teilchengröße der Goldteilchen der Nano-Gold-Katalysatoren ist vorzugsweise 1-50 nm, besonders bevorzugt 2-20 nm und insbesondere bevorzugt 4-10 nm.

Als Basen können Carbonate, Hydroxide oder Oxide in dem erfindungsgemäßen Verfahren verwendet werden. Bevorzugt sind die Hydroxide BOH.

Das erfindungsgemäße Verfahren wird vorzugsweise in Wasser durchgeführt.

Die Oxidationsreaktion wird bei einer Temperatur von 10 bis 80°C, bevorzugt zwischen 30 und 70°C, besonders bevorzugt zwischen 40 und 65°C, durchgeführt.

Der pH-Wert bei der Oxidation liegt bevorzugt zwischen 8 und 13, besonders bevorzugt zwischen 9 und 12,5.

Der Druck bei der Oxidationsreaktion ist vorzugsweise im Vergleich zu Atmosphärendruck erhöht.

Bei der Reaktion im alkalischen Medium entstehen zunächst die Alkalisalze (B = Li, Na, K, Rb, Cs) der acylierten Glycine mit 2 bis 22 Kohlenstoffatomen im Acylrest, bevorzugt mit 8 bis 18 Kohlenstoffatomen, bevorzugt die Natrium- oder Kaliumsalze. Besonders bevorzugt ist das Verfahren für Natriumcocoylglycinat und Kaliumcocoylglycinat. Durch Ansäuern mit anorganischen Säuren kann aus den Lösungen dann das acylierte Glycin erhalten werden. Bevorzugte Säuren sind Salz- und Schwefelsäure.

In einer weiteren Ausführungsform der Erfindung wurde der Tatsache Rechnung getragen, dass längerkettige (≥ C₈) Fettsäuremonoethanolamide, d.h. Fettsäuremonoethanolamide mit 8 oder mehr Kohlenstoffatomen im Acylrest R¹CO-, speziell Laurinsäuremonoethanolamid und Cocosfettsäuremonoethanolamid, für eine ausreichende Oxidationsreaktion ohne Zusatz geeigneter Lösemittel nicht ausreichend im Reaktionsmedium Wasser löslich sind. Somit würde der Vorteil der Kochsalzfreien Produktion der Zielsubstanzen in diesem Fall durch den zusätzlichen Einsatz von Lösemittel wieder teilweise aufgehoben.

Es wurde nun gefunden, dass Fettsäuremonoethanolamide mit 8 oder mehr Kohlenstoffatomen im Acylrest R'CO-, speziell Laurinsäuremonoethanolamid und Cocosfettsäuremonoethanolamid, in Lösungen von Alkalisalzen der Acylglycinate bzw. Fettsäureglycinate, insbesondere der Alkalisalze von Laurinsäure- oder Cocosfettsäureglycinaten, löslich sind. Dadurch ergibt sich eine elegante, lösemittelfreie Reaktionsführung dadurch, dass man eine Lösung von Fettsäuremonoethanolamid im Zielprodukt Acylglycinatsalz, vorzugsweise Natriumacylglycinat, herstellt (dies kann durch Rückmischung der fertigen Reaktionslösung mit Fettsäuremonoethanolamid erfolgen) und diese Mischung der katalytischen Oxidation unterwirft. Dabei wird das enthaltene Monoethanolamid oxidiert und wiederum eine Lösung eines Alkalisalzes (B = Li, Na, K, Rb, Cs) der Acylgylcinsäure der Formel (III) in Wasser erzeugt. Besonders bevorzugt sind hierbei die Natrium- und Kaliumsalze (B = Na, K).

Anschließend kann wiederum aus der alkalischen Reaktionslösung mittels geeigneter Säuren die Acylglycinsäure der Formel (III) freigesetzt werden. Bevorzugte Säuren sind Salzsäure und Schwefelsäure.

In einer bevorzugten Ausführungsform der Erfindung wird somit eine Lösung enthaltend ein oder mehrere Fettsäuremonoethanolamide der Formel (I) und ein oder mehrere Acylglycinate der Formel (II) der Oxidation unterworfen.

In dieser bevorzugten Ausführungsform der Erfindung werden die Fettsäuremonoethanolamide mittels Sauerstoff und heterogener Übergangsmetallkatalysatoren, bevorzugt heterogener Goldkatalysatoren, im alkalischen Medium zu Lösungen von Acylglycinaten oxidiert, wobei vor Beginn der Oxidationsreaktion eine Lösung des Fettsäuremonoethanolamids in einem Alkalisalz eines Acylglycinats vorliegt und diese Mischung in Wasser der Oxidationsreaktion unterworfen wird.

In dieser bevorzugten Ausführungsform der Erfindung beträgt das Massenverhältnis zwischen Fettsäuremonoethanolamid der Formel (I) und Acylglycinat der Formel (II) zu Beginn der Reaktion zwischen und 1 : 10 und 3:1, bevorzugt zwischen 1 : 2 und 2:1. Der Gesamtmassenanteil an Fettsäuremonoethanolamid der Formel (I) und Acylglycinat der Formel (II) beträgt zwischen 15 und 50 %, bevorzugt zwischen 20 und 40 %, besonders bevorzugt zwischen 25 und 35 %.

Das erfindungsgemäße Verfahren ergibt vorzugsweise Lösungen von Acylglycinaten der Formel (II) mit nur noch geringen Restgehalten an Fettsäuremonoethanolamid von < 10 Gew.-%, bevorzugt < 5 Gew.-%, besonders bevorzugt < 2 Gew.-%.

Die folgenden Beispiele illustrieren die Erfindung:

### Beispiel 1: Verfahren zur Herstellung von Glycinaten unter Verwendung von Goldkatalysatoren

In einen 2-Liter-Druckautoklaven mit Begasungsrührer werden 1 Liter einer wässrigen Lösung, enthaltend 15 Gew-% Cocosfettsäuremonoethanolamid und 15 Gew.-% Natriumcocoylglycinat, gegeben. Diese Mischung ist bis 80°C klar und flüssig. Die Menge an Natriumcocoylglycinat kann aus einem vorausgegangenen Ansatz entnommen werden bzw. aus einem vorausgegangenen Oxidationsansatz im Reaktor verbleiben. Nach Zugabe von 5 g eines Nanogoldkatalysators (1 Gew.-% Gold auf Titandioxid, Teilchengröße 4-8 nm) wird die Suspension mit Natronlauge auf pH 12 eingestellt und auf 60°C aufgeheizt. Nach Erreichen der Reaktionstemperatur wird die Reaktionslösung mit Sauerstoff auf einen Druck von 9 bar aufgepresst und durch Nachpressen auf diesem Druck gehalten. Während der gesamten Reaktionszeit wird mittels eines Autotitrators der pH-Wert der Mischung mit Natronlauge auf 12 gehalten. Nach 10 Stunden wird der Reaktor abgekühlt, entspannt und der Katalysator durch Filtration von der Reaktionslösung abgetrennt. Die Lösung zeigt einen Restgehalt von < 2 Gew.-% Cocosmonoethanolamid und ca. 32 Gew.-% Natriumcocoylglycinat.

### Beispiel 2: Verfahren zur Herstellung von Glycinaten unter Verwendung von Goldkatalysatoren

In einen 2-Liter-Druckautoklaven mit Begasungsrührer werden 1 Liter einer wässrigen Lösung, enthaltend 15 Gew.-% Cocosfettsäuremonoethanolamid und 15 Gew.-% Kaliumcocoylglycinat, gegeben. Diese Mischung ist bis 80 °C klar und flüssig. Die Menge an Kaliumcocoylglycinat kann aus einem vorausgegangenen Ansatz entnommen werden bzw. aus einem vorausgegangenen Oxidationsansatz im Reaktor verbleiben. Nach Zugabe von 5 g eines Nanogoldkatalysators (1 Gew.-% Gold auf Titandioxid, Teilchengröße 4-8 nm) wird die Suspension mit Natronlauge auf pH 12 eingestellt und auf 60° C aufgeheizt. Nach Erreichen der Reaktionstemperatur wird die Reaktionslösung mit Sauerstoff auf einen Druck von 9 bar aufgepresst und durch Nachpressen auf diesem Druck gehalten. Während der gesamten Reaktionszeit wird mittels eines Autotitrators der pH-Wert der Mischung mit Kalilauge auf 12 gehalten. Nach 10 Stunden wird der Reaktor abgekühlt, entspannt und der Katalysator durch Filtration von der Reaktionslösung abgetrennt. Die Lösung zeigt einen Restgehalt von < 1 Gew.-% Cocosmonoethanolamid und ca. 33 Gew.-% Kaliumcocoylglycinat.

## Patentansprüche

1. Verfahren zur Herstellung von Acylglycinatsalzen der Formel (II) 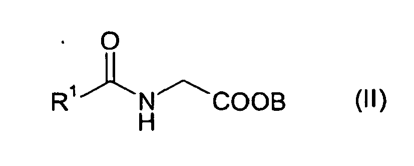 worin
R¹ einen gesättigten linearen oder verzweigten Alkylrest mit 1 bis 21 Kohlenstoffatomen oder einen ein- oder mehrfach ungesättigten linearen oder verzweigten Alkenylrest mit 2 bis 21 Kohlenstoffatomen und
B ein Kation, das von einer Base abgeleitet ist,
bedeutet, und/oder der entsprechenden protonierten Acylglycinsäuren, **dadurch gekennzeichnet, dass** ein oder mehrere Fettsäuremonoethanolamide der Formel (I) 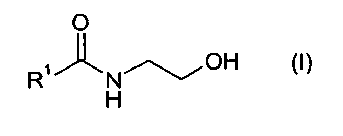 worin R¹ die oben angegebene Bedeutung besitzt,
mit Sauerstoff in Gegenwart eines Nebengruppenmetallkatalysators im alkalischen Medium zu einem oder mehreren Acylglycinatsalzen der Formel (II) oxidiert werden und im Fall der Herstellung der protonierten Acylglycinsäuren das oder die Acylglycinatsalze der Formel (II) zusätzlich mit einer Säure umgesetzt werden.

2. Verfahren nach Anspruch 1, **dadurch gekennzeichnet, dass** ein oder mehrere Fettsäuremonoethanolamide der Formel (I), worin der oder die Reste R¹ gesättigte lineare oder verzweigte Alkylreste mit 7 bis 17 Kohlenstoffatomen oder ein- oder mehrfach ungesättigte lineare oder verzweigte Alkenylreste mit 7 bis 17 Kohlenstoffatomen sind, in der Reaktion verwendet werden.

3. Verfahren nach Anspruch 1 oder 2, **dadurch gekennzeichnet, dass** das oder die Fettsäuremonoethanolamide der Formel (I) ausgewählt sind aus Laurinsäuremonoethanolamid, Myristinsäuremonoethanolamid, Caprylsäuremonoethanolamid, Caprinsäuremonoethanolamid, Palmitinsäuremonoethanolamid, Stearinsäuremonoethanolamid, Isostearinsäuremonoethanolamid und Cocosfettsäuremonoethanolamid.

4. Verfahren nach einem oder mehreren der Ansprüche 1 bis 3, **dadurch gekennzeichnet, dass** der Nebengruppenmetallkatalysator ein Cu, Ag, Au, Ru, Rh, Pd, Os, Ir, Pt-Katalysator oder deren Mischungen ist.

5. Verfahren nach einem oder mehreren der Ansprüche 1 bis 4, **dadurch gekennzeichnet, dass** der Nebengruppenmetallkatalysator ein Gold-Katalysator ist.

6. Verfahren nach Anspruch 5, **dadurch gekennzeichnet, dass** der Nebengruppenmetallkatalysator ein Nano-Gold-Katalysator ist.

7. Verfahren nach Anspruch 6, **dadurch gekennzeichnet, dass** der Nano-Gold-Katalysator auf einem oxidischen Träger aufgebracht ist.

8. Verfahren nach Anspruch 7, **dadurch gekennzeichnet, dass** der oxidische Träger aus Titandioxid besteht.

9. Verfahren nach einem oder mehreren der Ansprüche 6 bis 8, **dadurch gekennzeichnet, dass** der Nano-Gold-Katalysator 0,1 bis 5 Gew.-% Gold, bevorzugt 0,5 bis 2 Gew.-% Gold, enthält.

10. Verfahren nach einem oder mehreren der Ansprüche 6 bis 9, **dadurch gekennzeichnet, dass** die Teilchengröße der Goldteilchen des Nano-Gold-Katalysators von 1 bis 50 nm ist.

11. Verfahren nach einem oder mehreren der Ansprüche 1 bis 10, **dadurch gekennzeichnet, dass** eine Lösung enthaltend ein oder mehrere Fettsäuremonoethanolamide der Formel (I) und ein oder mehrere Acylglycinate der Formel (II) der Oxidation unterworfen werden.

## Claims

1. A process for preparing acylglycinate salts of the formula (II) 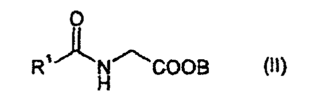 where
R¹ is a saturated linear or branched alkyl radical having from 1 to 21 carbon atoms or a monounsaturated or polyunsaturated linear or branched alkenyl radical having from 2 to 21 carbon atoms and
B is a cation derived from a base,
and/or the corresponding protonated acylglycine acids, which comprises oxidizing one or more fatty acid monoethanolamides of the formula (I) 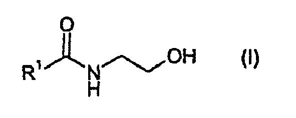 where R¹ is as defined above,
by means of oxygen in the presence of a transition metal catalyst in alkaline medium to form one or more acylglycinate salts of the formula (II) and, in the case of the preparation of the protonated acylglycine acids, additionally reacting the acylglycinate salt or salts of the formula (II) with an acid.

2. The process as claimed in claim 1, wherein one or more fatty acid monoethanolamides of the formula (I) in which the radicals R¹ are saturated linear or branched alkyl radicals having from 7 to 17 carbon atoms or monounsaturated or polyunsaturated linear or branched alkenyl radicals having from 7 to 17 carbon atoms are used in the reaction.

3. The process as claimed in claim 1 or 2, wherein the fatty acid monoethanolamide or monoethanolamides of the formula (I) is/are selected from among lauric acid monoethanolamide, myristic acid monoethanolamide, caprylic acid monoethanolamide, capric acid monoethanolamide, palmitic acid monoethanolamide, stearic acid monoethanolamide, isostearic acid monoethanolamide and coconut fatty acid monoethanolamide.

4. The process as claimed in one or more of claims 1 to 3, wherein the transition metal catalyst is a Cu, Ag, Au, Ru, Rh, Pd, Os, Ir, Pt catalyst or a mixture thereof.

5. The process as claimed in one or more of claims 1 to 4, wherein the transition metal catalyst is a gold catalyst.

6. The process as claimed in claim 5, wherein the transition metal catalyst is a nanogold catalyst.

7. The process as claimed in claim 6, wherein the nanogold catalyst has been applied to an oxidic support.

8. The process as claimed in claim 7, wherein the oxidic support comprises titanium dioxide.

9. The process as claimed in one or more of claims 6 to 8, wherein the nanogold catalyst contains from 0.1 to 5% by weight of gold, preferably from 0.5 to 2% by weight of gold.

10. The process as claimed in one or more of claims 6 to 9, wherein the particle size of the gold particles of the nanogold catalyst is from 1 to 50 nm.

11. The process as claimed in one or more of claims 1 to 10, wherein a solution comprising one or more fatty acid monoethanolamides of the formula (I) and one or more acylglycinates of the formula (II) is subjected to the oxidation.

## Revendications

1. Procédé pour la préparation de sels de type acylglycinate de formule (II) 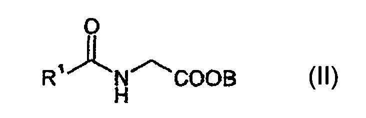 dans laquelle
R¹ représente un radical alkyle saturé linéaire ou ramifié ayant de 1 à 21 atomes de carbone ou un radical alcényle une ou plusieurs fois insaturé, linéaire ou ramifié, ayant de 2 à 21 atomes de carbone et
B est un cation qui est dérivé d'une base,
et/ou des acides acylglyciniques protonés correspondants, **caractérisé en ce qu'**on oxyde un ou plusieurs monoéthanolamides d'acides gras de formule (I) 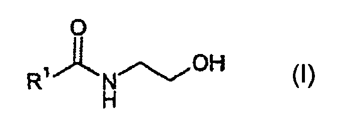 dans laquelle R¹ a la signification indiquée plus haut,
avec de l'oxygène en présence d'un catalyseur contenant un métal des groupes B en milieu alcalin, en un ou plusieurs sels de type acylglycinate de formule (II) et, dans le cas de la préparation des acides acylglyciniques protonés on fait en outre réagir avec un acide le ou les sels de type acylglycinate de formule (II).

2. Procédé selon la revendication 1, **caractérisé en ce que** dans la réaction on utilise un ou plusieurs monoéthanolamides d'acides gras de formule (I), dans laquelle le ou les radicaux R¹ sont des radicaux alkyle saturés linéaires ou ramifiés ayant de 7 à 17 atomes de carbone ou des radicaux alcényle une ou plusieurs fois insaturés, linéaires ou ramifiés, ayant de 7 à 17 atomes de carbone.

3. Procédé selon la revendication 1 ou 2, **caractérisé en ce que** le ou les monoéthanolamides d'acides gras de formule (I) sont choisis parmi le monoéthanolamide d'acide laurique, le monoéthanolamide d'acide myristique, le monoéthanolamide d'acide caprylique, le monoéthanolamide d'acide caprique, le monoéthanolamide d'acide palmitique, le monoéthanolamide d'acide stéarique, le monoéthanolamide d'acide isostéarique et le monoéthanolamide d'acide gras de coco.

4. Procédé selon une ou plusieurs des revendications 1 à 3, **caractérisé en ce que** le catalyseur contenant un métal des groupes B est un catalyseur à base de Cu, Ag, Au, Ru, Rh, Pd, Os, Ir, Pt ou des mélanges de ceux-ci.

5. Procédé selon une ou plusieurs des revendications 1 à 4, **caractérisé en ce que** le catalyseur contenant un métal des groupes B est un catalyseur à l'or.

6. Procédé selon la revendication 5, **caractérisé en ce que** le catalyseur contenant un métal des groupes B est un catalyseur au nano-or.

7. Procédé selon la revendication 6, **caractérisé en ce que** le catalyseur au nano-or est appliqué sur un support de type oxyde.

8. Procédé selon la revendication 7, **caractérisé en ce que** le support de type oxyde consiste en dioxyde de titane.

9. Procédé selon une ou plusieurs des revendications 6 à 8, **caractérisé en ce que** le catalyseur au nano-or contient de 0,1 à 5 % en poids d'or, de préférence de 0,5 à 2 % en poids d'or.

10. Procédé selon une ou plusieurs des revendications 6 à 9, **caractérisé en ce que** la taille de particule des particules d'or du catalyseur au nano-or est de 1 à 50 nm.

11. Procédé selon une ou plusieurs des revendications 1 à 10, **caractérisé en ce qu'**on soumet à l'oxydation un ou plusieurs monoéthanolamides d'acides gras de formule (I) et un ou plusieurs acylglycinates de formule (II).
